# EUROPEAN PATENT SPECIFICATION

(11) **EP 3 700 623 B1**
(45) Date of publication and mention of the grant of the patent: **31.07.2024**
(21) Application number: 18801138.1
(22) Date of filing: 23.10.2018
(51) Int. Cl.: A61N 1/36, A61B 5/0538, A61B 5/00, A61B 5/021, A61B 5/0245, A61B 5/026, A61B 5/145, A61N 1/20, A61N 1/05

(54) **ELECTRIC FIELD CANCER THERAPY DEVICES WITH FEEDBACK MECHANISMS AND DIAGNOSTICS**
KREBSTHERAPIEVORRICHTUNGEN MIT ELEKTRISCHEM FELD UND RÜCKKOPPLUNGSMECHANISMEN SOWIE DIAGNOSE
DISPOSITIFS DE TRAITEMENT DU CANCER PAR CHAMP ÉLECTRIQUE COMPRENANT DES MÉCANISMES DE RÉTROACTION ET DES DIAGNOSTICS

(30) Priority: 23.10.2017 US 201762575748 P; 22.10.2018 US 201816167140
(43) Date of publication of application: 02.09.2020
(73) Proprietor: Cardiac Pacemakers, Inc., St. Paul, Minnesota 55112 (US)
(72) Inventor: SCHMIDT, Brian L., White Bear Lake Minnesota 55110 (US); LUDWIG, Jacob M., Isanti Minnesota 55040 (US); HAASL, Benjamin J., Forest Lake Minnesota 55025 (US); KANE, Michael J., St. Paul Minnesota 55105 (US)
(74) Representative: Vossius & Partner Patentanwälte Rechtsanwälte mbB
(86) International application number: PCT/US2018/057127
(87) International publication number: WO 2019/084021

(56) References cited:
- WO-A1-2016/199142
- US-A1- 2005 096 584
- US-A1- 2011 137 229
- US-A1- 2012 203 307
- US-A1- 2017 215 939
- US-A1- 2017 266 371

## Description

### Field

Embodiments herein relate to medical device systems including electric field shaping elements for use in treating cancerous tumors within a bodily tissue. More specifically, embodiments herein relate to using electric field shaping elements configured to redirect or concentrate therapeutic electric fields at the site of a cancerous tumor.

### Background

According to the American Cancer Society, cancer accounts for nearly 25% of the deaths that occur in the United States each year. The current standard of care for cancerous tumors can include first-line therapies such as surgery, radiation therapy, and chemotherapy. Additional second-line therapies can include radioactive seeding, cryotherapy, hormone or biologics therapy, ablation, and the like. Combinations of first-line therapies and second-line therapies can also be a benefit to patients if one particular therapy on its own is not effective.

Cancerous tumors can form if one normal cell in any part of the body mutates and then begins to grow and multiply too much and too quickly. Cancerous tumors can be a result of a genetic mutation to the cellular DNA or RNA that arises during cell division, an external stimulus such as ionizing or non-ionizing radiation, exposure to a carcinogen, or a result of a hereditary gene mutation. Regardless of the etiology, many cancerous tumors are the result of unchecked rapid cellular division.

Mitosis is the process of cellular division that is a part of the cell cycle for all somatic cells in the body, including many types of cancerous cells. Mitosis includes four basic phases: prophase, metaphase, anaphase, and telophase. Just prior to prophase, a cell will copy its chromosomes to create two identical sister chromatids. During prophase, the chromosomes start to condense and the nuclear membrane surrounding the nucleus disappears. The mitotic spindle also begins to form during prophase. The mitotic spindle includes a self-organized bipolar array of microtubules and centrosomes. Microtubules are generally formed from the polymerization of the highly polar alpha-tubulin and beta-tubulin proteins. Centrosomes are similarly protein-based organelles, two of which migrate to opposite sides of the dividing cell at this phase. The negatively charged end of the microtubules attach to the centrosomes. The positively charged end of the microtubules radiate toward the equator of the dividing cell where they eventually attach to a kinetochore of each sister chromatid. Metaphase can be defined by all chromosomes being aligned at the equator of the dividing cell and bound in the mitotic spindle. An equal number of sister chromatids are then pulled toward opposite ends of the cell during anaphase. Once all chromosomes have been separated, the process of telophase begins, where the cell membrane begins to form a cleavage furrow between the two newly forming sister cells, and cell division becomes complete once the cells physically separate from one another in a process called cytokinesis.

US 2005/0096584 A1 discloses implantable medical devices and methods for electroporation treatment of subcutaneous tumors.

US 2017/0215939 A1 discloses an apparatus that includes a device for measuring the tumor impedance, an AC signal generator with a controllable output frequency, a processor for estimating the size of tumor cells and setting the frequency of the AC signal generator based thereon, and at least one pair of electrodes operatively connected to the AC signal generator such that an alternating electric field is applied to the tumor.

US 2012/0203307 A1 discloses a device for electrical treatment of malignant tumors and neoplasms by applying a voltage to affected tissue.

WO 2016/199142 A1 discloses an implantable device for monitoring a condition of a biological tissue. The device comprises a sensor comprising a plurality of electrodes spaced apart from each other, an electric signal source configured to provide an electric signal to one or more pairs of neighboring or non-neighboring electrodes of said plurality of electrodes, and an electric signal measurement unit configured to measure impedance values between each of said one or more pairs of electrodes, wherein said signals produced by said electric signal measurement unit are indicative of a characteristic of a biological tissue adjacent the pair of electrodes.

### Summary

The claimed invention is concerned with an implantable medical device for treating a cancerous tumor of a patient as defined by the appended independent claim 1. Further embodiments of the claimed invention are described in the appended dependent claims.

Also disclosed, but not part of the claimed invention, is a method for treating a cancerous tumor. The method can include implanting one or more electrodes within a patient, and measuring the impedance of tissue within the patient along a vector passing through or near a cancerous tumor. The method can also include administering an electric field to the cancerous tumor of the patient based on the measured impedance.

The method can include administering the electric field to the cancerous tumor of the patient based on changes in the measured impedance.

The method can include using electrodes that include electric field generating electrodes and passive electric field sensing electrodes.

The method can include using passive electric field sensing electrodes that are implanted within the cancerous tumor.

The method can include using electrical field generating electrodes that are implanted adjacent to the cancerous tumor.

Further disclosed herein, but also not part of the claimed invention, is a method for treating a cancerous tumor. The method can include implanting one or more electrodes within a patient and measuring the impedance of tissue within the patient along a vector passing through or near a cancerous tumor. The method can also include assessing tumor progression based on the measured impedance.

The method can include administering an electric field to the cancerous tumor of the patient based on the measured impedance.

A decreased impedance can be indicative of tumor progression.

The measured impedance can include a measured low frequency impedance.

The low frequency can include a frequency of about 1 Hz to about 10 Hz.

The measured impedance can include a measured high frequency impedance.

The high frequency can include a frequency of about 10 kHz to about 1 MHz.

The high frequency can include a frequency of about 100 kHz to about 300 kHz.

Further disclosed yet, but also not part of the claimed invention, is a method for treating a cancerous tumor. The method can include implanting one or more electric field generating electrodes within a patient and implanting one or more electric field sensing electrodes within the patient. The method can further include delivering an electric field from the electric field generating electrodes to a cancerous tumor and measuring an electric field strength with the electric field sensing electrodes in or around the cancerous tumor. The method can further include adjusting the delivered electric field to a desired electric field strength based on the measured electrical field strength.

Also disclosed, but again not part of the claimed invention, is a method for treating a cancerous tumor. The method can include implanting one or more electrodes within a patient and measuring a property of an electric field delivered along a vector passing through or near a cancerous tumor, the property selected from the group consisting of impedance, capacitance, and electric field strength. The method can also include delivering of an electric field to the cancerous tumor based on the measured property.

The measured property can include impedance.

The measured property can include capacitance.

The measured property can include field strength.

The method can include adjusting the delivered electric field based on the measured property.

Adjusting the delivered electric field can include one or more of changing the electric field strength, changing the waveform of the electric field, and changing the frequency of the electric field.

Further disclosed, but not part of the claimed invention, is a method for treating a cancerous tumor. The method can include implanting one or more electric field generating electrodes within a patient and implanting an evoked potential sensor within the patient. The method can include delivering an electric field from the electric field generating electrodes to a cancerous tumor. The method can include monitoring for an evoked potential using the evoked potential sensor to determine whether or not the delivered electric field strength is above a predetermined threshold for neural or muscular recruitment and reducing the strength of the electric field if the electric field strength is above a threshold for neural or muscular recruitment.

Still further disclosed, but not part of the claimed invention, is a method for treating a cancerous tumor. The method can include implanting one or more electric field generating electrodes within a patient and measuring a heart rate of the patient. The method can include delivering an electric field from the electric field generating electrodes to a cancerous tumor. The method can include monitoring for changes in the heart rate of the patient in response to the delivered electric field and adjusting the electric field if changes in the heart rate are detected.

Adjusting the electric field can include reducing the strength of the delivered electric field to a predetermined threshold.

Adjusting the electric field can include increasing the strength of the delivered electric field to a predetermined threshold.

Also disclosed, but again not part of the claimed invention, is a method for treating a cancerous tumor. The method can include implanting one or more electric field generating electrodes within a patient and delivering an electric field from the electric field generating electrodes to a cancerous tumor. The method can include measuring at least one property selected from the group consisting of temperature, blood flow, blood pressure, metabolite concentrations, and systemic cancerous marker concentrations.

The method can include monitoring for changes in the at least one property.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. As already mentioned, the scope of the claimed invention is defined by the appended claims and their legal equivalents.

### Brief Description of the Figures

Aspects may be more completely understood in connection with the following drawings, in which:
FIG. 1 is a schematic view of a medical system in accordance with various embodiments herein.
FIG. 2 is a schematic view of a medical system in accordance with various embodiments herein.
FIG. 3 is a schematic cross-sectional view of a medical device in accordance with various embodiments herein.
FIG. 4 is a schematic view of a leadless medical device.
FIG. 5 is a schematic diagram of components of a medical device in accordance with various embodiments herein.
FIG. 6 is a schematic view of a medical device in accordance with various embodiments herein.
FIG. 7 is a schematic view of a lead in accordance with various embodiments herein.
FIG. 8 is a flow chart of a method of treating a cancerous tumor.
FIG. 9 is a flow chart of a method of treating a cancerous tumor.
FIG. 10 is a flow chart of a method of treating a cancerous tumor.
FIG. 11 is a flow chart of a method of treating a cancerous tumor.
FIG. 12 is a flow chart of a method of treating a cancerous tumor.
FIG. 13 is a flow chart of a method of treating a cancerous tumor.
FIG. 14 is a flow chart of a method of treating a cancerous tumor.
FIG. 15 is a plot of an exemplary therapy parameter in accordance with various embodiments herein.

While embodiments are susceptible to various modifications and alternative forms, specifics thereof have been shown by way of example and drawings, and will be described in detail. It should be understood, however, that the scope of the claimed invention is not limited to the particular embodiments described, but is defined by the appended set of claims, with the intention to cover modifications, legal equivalents, and alternatives falling within the scope of the appended set of claims.

### Detailed Description

As referenced above, many cancerous tumors can result from unchecked rapid cellular division. Some traditional first-line therapies to treat cancerous tumors can include surgery, radiation therapy, and chemotherapy. However, many first-line therapies have undesirable concomitant side effects, such as fatigue, hair loss, immunosuppression, and long surgical recovery times, to name a few.

While not intending to be bound by theory, it is believed that alternating electric fields can disrupt mitosis within a cancerous tumor by interfering with the dipole alignment of key proteins involved in cellular division; tubulin and septin in particular. The polymerization of tubulin proteins that form microtubule spindle fibers can be disrupted, thus preventing the formation of spindle fibers required for chromosome separation. This can halt cellular division at the metaphase stage of mitosis. In some instances an alternating electric field can halt polymerization of already growing spindle fibers, leading to incomplete spindles and unequal chromosome separation during anaphase, should the cell survive that long. In each case, halting microtubule spindle formation and unequal chromosome separation during anaphase caused by incomplete polymerization of microtubules, can result in apoptosis (i.e., programmed cell death).

It is also believed that alternating electric fields can lead to increased electric field density near the cleavage furrow of the dividing cells during telophase. An increased electric field density in the region of the cleavage furrow can result in dielectrophoresis of charged macromolecules, such as proteins and nucleic acids, toward the high electric field density at the furrow. The unequal concentration of key macromolecules required for cellular division at the site of the cleavage furrow can disrupt the final separation of the sister cells during telophase and eventually lead to apoptosis.

Feedback obtained during electric field therapy can be used to monitor the effectiveness of treating a cancerous tumor with the therapy. Data can be measured for parameters such as impedance, capacitance, field strength, etc. to direct a particular course of treatment. Without being bound by any particular theory, it is believed that a cancerous tumor has a particular impedance associated therewith. The impedance associated with a tumor can change as the size or cellular makeup of the tumor changes. Therefore, impedance can be monitored during the course of an electric field therapy in order to determine if the cancerous tumor is responding to therapy. In some instances, an increase in impedance of the tissue in a treatment area including a cancerous tumor can be indicative of tumor regression. In other instances, a decrease or no observed change in impedance of the tissue in a treatment area can be indicative of tumor progression or lack of change in the tumor respectively. Other physiological properties associated with a cancerous tumor, such as blood flow, metabolite concentrations, systemic cancer markers, and temperature can also be used in conjunction with impedance analysis to monitor the progression or regression of a cancerous tumor in response to electric field therapy.

Therapy parameters including, but not limited to, one or more of the amplitude, frequency, pulse width, waveform, directionality, vector, and/or duty cycle of the electric field therapy can be modulated and/or changed, tuned or otherwise altered based on measured values for at least one of impedance, capacitance, field strength, etc.

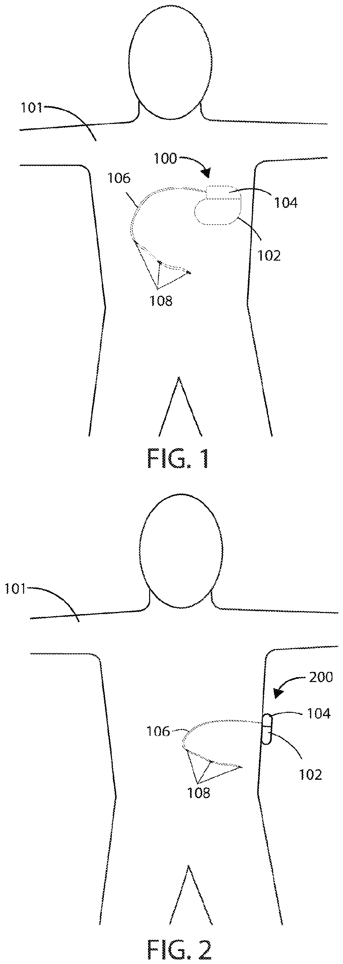

Referring now to FIG. 1, a schematic view is shown of a medical device 100 in accordance with various embodiments herein. The medical device 100 can be implanted entirely within the body of a patient 101 at or near the site of a cancerous tumor located within a bodily tissue. Various implant sites can be used including areas such as in the limbs, the upper torso, the abdominal area, the head, and the like.

Referring now to FIG. 2, another schematic view is shown of a medical device 200 in accordance with various embodiments herein. The medical device 200 can be partially implanted within the body of a patient 101. In some embodiments, the medical device can be partially implanted and partially external to the body of a patient. In other embodiments, a partially implanted medical device can include a transcutaneous connection between components disposed internal to the body and external to the body. A partially implanted medical device can wirelessly communicate with a partially external portion of a medical device over a wireless connection.

In some embodiments, a portion of the medical device can be entirely implanted and a portion of the medical device can be entirely external. For example, in some embodiments, one or more electrodes or leads can be entirely implanted within the body, whereas the portion of the medical device that generates an electric field, such as an electric field generator, can be entirely external to the body. It will be appreciated that in some embodiments described herein, the electric field generators described can include the many of the same components as and can be configured to perform many of the same functions as a pulse generator. In embodiments where a portion of a medical device is entirely implanted and a portion of the medical device is entirely external, the portion of the medical device that is entirely external can communicate wirelessly with the portion of the medical device that is entirely internal. However, in other embodiments a wired connection can be used.

The medical device 100 or medical device 200 can include a housing 102 and a header 104 coupled to the housing 102. Various materials can be used. However, in some embodiments, the housing 102 can be formed of a material such as a metal, ceramic, polymer, composite, or the like. In some embodiments, the housing 102, or one or more portions thereof, can be formed of titanium. The header 104 can be formed of various materials, but in some embodiments the header 104 can be formed of a translucent polymer such as an epoxy material. In some embodiments the header 104 can be hollow. In other embodiments the header 104 can be filled with components and/or structural materials such as epoxy or another material such that it is non-hollow.

In some embodiments where a portion of the medical device 100 or 200 is partially external, the header 104 and housing 102 can be surrounded by a protective casing made of durable polymeric material. In other embodiments, where a portion of the medical device 100 or 200 is partially external, the header 104 and housing 102 can be surrounded by a protective casing made of a combination of polymeric material, metallic material, and/or glass material.

The header 104 can be coupled to one or more leads 106. The header 104 can serve to provide fixation of the proximal end of one or more leads 106 and electrically couple the one or more leads 106 to one or more components within the housing 102. The one or more leads 106 can include one or more electrodes 108 disposed along the length of the electrical leads 106. In some embodiments, electrodes 108 can include electric field generating electrodes and in other embodiments electrodes 108 can include electric field sensing electrodes. In some embodiments, leads 106 can include both electric field generating and electric field sensing electrodes. In other embodiments, leads 106 can include any number of electrodes that are both electric field sensing and electric field generating. It will be appreciated that while many embodiments of medical devices herein are designed to function with leads, leadless medical devices that generate electrical fields are also contemplated herein.

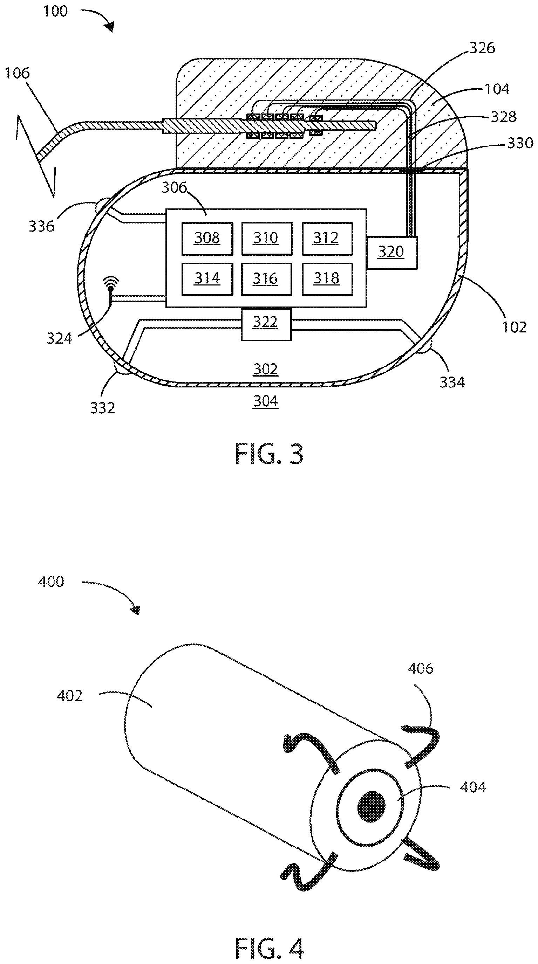

Referring now to FIG. 3, a schematic cross-sectional view of medical device 100 is shown in accordance with various embodiments herein. Housing 102 can define an interior volume 302 that can be hollow and that in some embodiments is hermetically sealed off from the area 304 outside of medical device 100. In other embodiments the housing 102 can be filled with components and/or structural materials such that it is non-hollow. The medical device 100 can include control circuitry 306, which can include various components 308, 310, 312, 314, 316, and 318 disposed within housing 102. In some embodiments, these components can be integrated and in other embodiments these components can be separate. In yet other embodiments, there can be a combination of both integrated and separate components. The medical device 100 can also include an antenna 324, to allow for unidirectional or bidirectional wireless data communication. In some embodiments, the components of medical device 100 can include an inductive energy receiver coil (not shown) communicatively coupled or attached thereto to facilitate transcutaneous recharging of the medical device via recharging circuitry.

The various components 308, 310, 312, 314, 316, and 318 of control circuitry 306 can include, but are not limited to, a microprocessor, memory circuit (such as random access memory (RAM) and/or read only memory (ROM)), recorder circuitry, controller circuit, a telemetry circuit, a power supply circuit (such as a battery), a timing circuit, and an application specific integrated circuit (ASIC), a recharging circuit, amongst others. Control circuitry 306 can be in communication with an electric field generating circuit 320 that can be configured to generate electric current to create one or more fields. The electric field generating circuit 320 can be integrated with the control circuitry 306 or can be a separate component from control circuitry 306. Control circuitry 306 can be configured to control delivery of electric current from the electric field generating circuit 320. In some embodiments, the electric field generating circuit 320 can be present in a portion of the medical device that is external to the body.

In some embodiments, the control circuitry 306 can be configured to direct the electric field generating circuit 320 to deliver an electric field using one or more frequencies selected from a range of between 10 kHz to 1 MHz. In some embodiments, the control circuitry 306 can be configured to direct the electric field generating circuit 320 to deliver an electric field at one or more frequencies selected from a range of between 100 kHz to 500 kHz. In some embodiments, the control circuitry 306 can be configured to direct the electric field generating circuit 320 to deliver an electric field at one or more frequencies selected from a range of between 100 kHz to 300 kHz. In some embodiments, the control circuitry 306 can be configured to direct the electric field generating circuit 320 to periodically deliver an electric field using one or more frequencies greater than 1 MHz.

In some embodiments, the electric field can be effective in disrupting cellular mitosis in cancerous cells. The electric field can be delivered to the site of a cancerous tumor along more than one vector. In some examples, the electric field can be delivered along at least one vector, including at least one of the lead electrodes. In some embodiments, at least two vectors with spatial diversity between the two vectors can be used. The vectors can be spatially separated (e.g., the vectors can be disposed at an angle with respect to one another) by at least about 10, 20, 30, 40, 50, 60, 70, 80 or 90 degrees.

A desired electric field strength can be achieved by delivering an electric current between two electrodes. The specific current and voltage at which the electric field is delivered can vary and can be adjusted to achieve the desired electric field strength at the site of the tissue to be treated. In some embodiments, the control circuitry 306 can be configured to direct the electric field generating circuit 320 to deliver an electric field using currents ranging from 1 mAmp to 1000 mAmp to the site of a cancerous tumor. In some embodiments, the control circuitry 306 can be configured to direct the electric field generating circuit 320 to deliver an electric field using currents ranging from 20 mAmp to 500 mAmp to the site of a cancerous tumor. In some embodiments, the control circuitry 306 can be configured to direct the electric field generating circuit 320 to deliver an electric field using currents ranging from 30 mAmp to 300 mAmp to the site of a cancerous tumor.

In some embodiments, the control circuitry 306 can be configured to direct the electric field generating circuit 320 to deliver an electric field using currents including 1 mAmp, 2 mAmp, 3 mAmp, 4 mAmp, 5 mAmp, 6 mAmp, 7 mAmp, 8 mAmp, 9 mAmp, 10 mAmp, 15 mAmp, 20 mAmp, 25 mAmp, 30 mAmp, 35 mAmp, 40 mAmp, 45 mAmp, 50 mAmp, 60 mAmp, 70 mAmp, 80 mAmp, 90 mAmp, 100 mAmp, 125 mAmp, 150 mAmp, 175 mAmp , 200 mAmp, 225 mAmp, 250 mAmp, 275 mAmp, 300 mAmp, 325 mAmp, 350 mAmp, 375 mAmp, 400 mAmp, 425 mAmp, 450 mAmp, 475 mAmp, 500 mAmp, 525 mAmp, 550 mAmp, 575 mAmp, 600 mAmp, 625 mAmp, 650 mAmp, 675 mAmp, 700 mAmp, 725 mAmp, 750 mAmp, 775 mAmp, 800 mAmp, 825 mAmp, 850 mAmp, 875 mAmp, 900 mAmp, 925 mAmp, 950 mAmp, 975 mAmp, or 1000 mAmp. It will be appreciated that the control circuitry can be configured to direct the electric field generating circuit 320 to deliver an electric field at a current falling within a range, wherein any of the forgoing currents can serve as the lower or upper bound of the range, provided that the lower bound of the range is a value less than the upper bound of the range.

In some embodiments, the control circuitry 306 can be configured to direct the electric field generating circuit 320 to deliver an electric field using voltages ranging from 1 Vᵣₘₛ to 50 Vᵣₘₛ to the site of a cancerous tumor. In some embodiments, the control circuitry 306 can be configured to direct the electric field generating circuit 320 to deliver an electric field using voltages ranging from 5 Vᵣₘₛ to 30 Vᵣₘₛ to the site of a cancerous tumor. In some embodiments, the control circuitry 306 can be configured to direct the electric field generating circuit 320 to deliver an electric field using voltages ranging from 10 Vᵣₘₛ to 20 Vᵣₘₛ to the site of a cancerous tumor.

In some embodiments, the control circuitry 306 can be configured to direct the electric field generating circuit 320 to deliver an electric field using one or more voltages including 1 Vᵣₘₛ, 2 Vᵣₘₛ, 3 Vᵣₘₛ, 4 Vᵣₘₛ, 5 Vᵣₘₛ, 6 Vᵣₘₛ, 7 Vᵣₘₛ, 8 Vᵣₘₛ, 9 Vᵣₘₛ, 10 Vᵣₘₛ, 15 Vᵣₘₛ, 20 Vᵣₘₛ, 25 Vᵣₘₛ, 30 Vᵣₘₛ, 35 Vᵣₘₛ, 40 Vᵣₘₛ, 45 Vᵣₘₛ, or 50 Vᵣₘₛ. It will be appreciated that the control circuitry can be configured to direct the electric field generating circuit 320 to deliver an electric field using a voltage falling within a range, wherein any of the forgoing voltages can serve as the lower or upper bound of the range, provided that the lower bound of the range is a value less than the upper bound of the range.

In some embodiments, the control circuitry 306 can be configured to direct the electric field generating circuit 320 to deliver and electric field using one or more frequencies including 10 kHz, 20 kHz, 30 kHz, 40 kHz, 50 kHz, 60 kHz, 70 kHz, 80 kHz, 90 kHz, 100 kHz, 125 kHz, 150 kHz, 175 kHz, 200 kHz, 225 kHz, 250 kHz, 275 kHz, 300 kHz, 325 kHz, 350 kHz, 375 kHz, 400 kHz, 425 kHz, 450 kHz, 475 kHz, 500 kHz, 525 kHz, 550 kHz, 575 kHz, 600 kHz, 625 kHz, 650 kHz, 675 kHz, 700 kHz, 725 kHz, 750 kHz, 775 kHz, 800 kHz, 825 kHz, 850 kHz, 875 kHz, 900 kHz, 925 kHz, 950 kHz, 975 kHz, 1 MHz. It will be appreciated that the electric field generating circuit 320 can deliver an electric field using a frequency falling within a range, wherein any of the foregoing frequencies can serve as the upper or lower bound of the range, provided that the upper bound is greater than the lower bound.

In some embodiments, the control circuitry 306 can be configured to direct the electric field generating circuit 320 to generate one or more applied electric field strengths selected from a range of between 0.25 V/cm to 1000 V/cm. In some embodiments, the control circuitry 306 can be configured to direct the electric field generating circuit 320 to generate one or more applied electric field strengths of greater than 3 V/cm. In some embodiments, the control circuitry 306 can be configured to direct the electric field generating circuit 320 to generate one or more applied electric field strengths selected from a range of between 1 V/cm to 10 V/cm. In some embodiments, the control circuitry 306 can be configured to direct the electric field generating circuit 320 to generate one or more applied electric field strengths selected from a range of between 3 V/cm to 5 V/cm.

In other embodiments, the control circuitry 306 can be configured to direct the electric field generating circuit 320 to generate one or more applied electric field strengths including 0.25 V/cm, 0.5 V/cm, 0.75 V/cm, 1.0 V/cm, 2.0 V/cm, 3.0 V/cm, 5.0 V/cm, 6.0 V/cm, 7.0 V/cm, 8.0 V/cm, 9.0 V/cm, 10.0 V/cm, 20.0 V/cm, 30.0 V/cm, 40.0 V/cm, 50.0 V/cm, 60.0 V/cm, 70.0 V/cm, 80.0 V/cm, 90.0 V/cm, 100.0 V/cm, 125.0 V/cm, 150.0 V/cm, 175.0 V/cm, 200.0 V/cm, 225.0 V/cm, 250.0 V/cm, 275.0 V/cm, 300.0 V/cm, 325.0 V/cm, 350.0 V/cm, 375.0 V/cm, 400.0 V/cm, 425.0 V/cm, 450.0 V/cm, 475.0 V/cm, 500.0 V/cm, 600.0 V/cm, 700.0 V/cm, 800.0 V/cm, 900.0 V/cm, 1000.0 V/cm. It will be appreciated that the electric field generating circuit 320 can generate an electric field having a field strength at a treatment site falling within a range, wherein any of the foregoing field strengths can serve as the upper or lower bound of the range, provided that the upper bound is greater than the lower bound.

In some embodiments, the control circuitry 306 can be configured to direct the electric field generating circuit 320 to deliver an electric field via leads 106 to the site of a cancerous tumor located within a bodily tissue. In other embodiments, the control circuitry 306 can be configured to direct the electric field generating circuit 320 to deliver an electric field via the housing 102 of medical device 100 to the site of a cancerous tumor located within a bodily tissue. In other embodiments, the control circuitry 306 can be configured to direct the electric field generating circuit 320 to deliver an electric field between leads 106 and the housing 102 of medical device 100. In some embodiments, one or more leads 106 can be in electrical communication with the electric field generating circuit 320. In some embodiments, the one or more leads 106 can include one or more electrodes 108 disposed along the length of the leads 106, where the electrodes 108 can be in electrical communication with the electric field generating circuit 320.

In some embodiments, various components within medical device 100 can include an electric field sensing circuit 322 configured to generate a signal corresponding to sensed electric fields. Electric field sensing circuit 322 can be integrated with control circuitry 306 or it can be separate from control circuitry 306.

Sensing electrodes can be disposed on or adjacent to the housing of the medical device, on one or more leads connected to the housing, on a separate device implanted near or in the tumor, or any combination of these locations. In some embodiments, the electric field sensing circuit 322 can include a first sensing electrode 332 and a second sensing electrode 334. In other embodiments, the housing 102 itself can serve as a sensing electrode for the electric field sensing circuit 322. The electrodes 332 and 334 can be in communication with the electric field sensing circuit 322. The electric field sensing circuit 322 can measure the electrical potential difference (voltage) between the first electrode 332 and the second electrode 334. In some embodiments, the electric field sensing circuit 322 can measure the electrical potential difference (voltage) between the first electrode 332 or second electrode 334, and an electrode disposed along the length of one or more leads 106. In some embodiments, the electric field sensing circuit can be configured to measure sensed electric fields and to record electric field strength in V/cm.

It will be appreciated that the electric field sensing circuit 322 can additionally measure an electrical potential difference between the first electrode 332 or the second electrode 334 and the housing 102 itself. In other embodiments, the medical device can include a third electrode 336, which can be an electric field sensing electrode or an electric field generating electrode. In some embodiments, one or more sensing electrodes can be disposed along lead 106 and can serve as additional locations for sensing an electric field. Many combinations can be imagined for measuring electrical potential difference between electrodes disposed along the length of one or more leads 106 and the housing 102 in accordance with the embodiments herein.

In some embodiments, the one or more leads 106 can be in electrical communication with the electric field generating circuit 320. The one or more leads 106 can include one or more electrodes 108, as shown in FIGS. 1 and 2. In some embodiments, various electrical conductors, such as electrical conductors 326 and 328, can pass from the header 104 through a feed-through structure 330 and into the interior volume 302 of medical device 100. As such, the electrical conductors 326 and 328 can serve to provide electrical communication between the one or more leads 106 and control circuitry 306 disposed within the interior volume 302 of the housing 102.

In some embodiments, recorder circuitry can be configured to record the data produced by the electric field sensing circuit 322 and record time stamps regarding the same. In some embodiments, the control circuitry 306 can be hardwired to execute various functions, while in other embodiments the control circuitry 306 can be directed to implement instructions executing on a microprocessor or other external computation device. A telemetry circuit can also be provided for communicating with external computation devices such as a programmer, a home-based unit, and/or a mobile unit (e.g. a cellular phone, personal computer, smart phone, tablet computer, and the like).

Referring now to FIG. 4, leadless medical device 400 is shown. The leadless medical device 400 can include a housing 402 and a header 404 coupled to the housing 402. Various materials can be used. However, in some embodiments, the housing 402 can be formed of a material such as a metal, ceramic, polymer, composite, or the like. In some embodiments, the housing 402, or one or more portions thereof, can be formed of titanium. The header 404 can be formed of various materials, but in some embodiments the header 404 can be formed of a translucent polymer such as an epoxy material. In some embodiments the header 404 can be hollow. In other embodiments the header 404 can be filled with components and/or structural materials such as epoxy or another material such that it is non-hollow. In some embodiments, leadless medical device 400 can include fixation elements 406 to keep a leadless medical device 400 positioned at or near the site of a cancerous tumor within the body. In some embodiments, fixation elements 406 can include talons, tines, helices, bias, and the like.

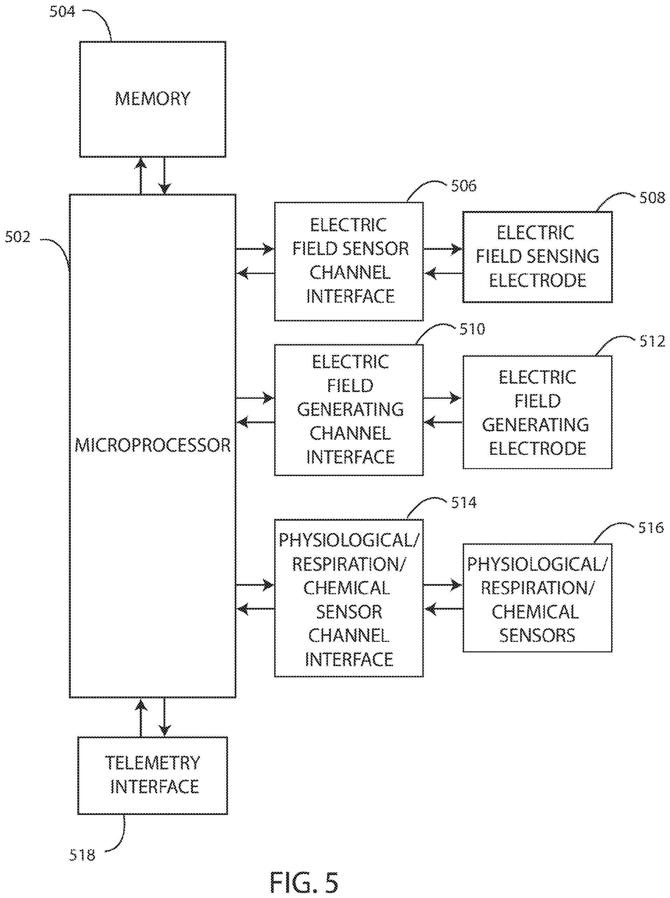

Elements of various embodiments of the medical devices described herein are shown in FIG. 5. However, it will be appreciated that some embodiments can include additional elements beyond those shown in FIG. 5. In addition, some embodiments may lack some elements shown in FIG. 5. The medical devices as embodied herein can gather information through one or more sensing channels and can output information through one or more field generating channels. A microprocessor 502 can communicate with a memory 504 via a bidirectional data bus. The memory 504 can include read only memory (ROM) or random access memory (RAM) for program storage and RAM for data storage. The microprocessor 502 can also be connected to a telemetry interface 518 for communicating with external devices such as a programmer, a home-based unit and/or a mobile unit (e.g. a cellular phone, personal computer, smart phone, tablet computer, and the like) or directly to the cloud or another communication network as facilitated by a cellular or other data communication network. In some embodiments, the medical device can include an inductive energy receiver coil interface (not shown) communicatively coupled or attached thereto to facilitate transcutaneous recharging of the medical device.

The medical device can include one or more electric field sensing electrodes 508 and one or more electric field sensor channel interfaces 506 that can communicate with a port of microprocessor 502. The medical device can also include one or more electric field generating electrodes 512 and one or more electric field generating channel interfaces 510 that can communicate with a port of microprocessor 502. The medical device can also include one or more physiological sensors, respiration sensors, or chemical sensors 516 and one or more physiological/respiration/chemical sensor channel interfaces 514 that can communicate with a port of microprocessor 502. The channel interfaces 506, 510, and 514 can include various components such as analog-to-digital converters for digitizing signal inputs, sensing amplifiers, registers which can be written to by the control circuitry in order to adjust the gain and threshold values for the sensing amplifiers, source drivers, modulators, demodulators, multiplexers, and the like.

In some embodiments, the physiological sensors can include sensors that monitor temperature, blood flow, blood pressure, and the like. In some embodiments, the respiration sensors can include sensors that monitor respiration rate, respiration peak amplitude, and the like. In some embodiments, the chemical sensors can measure the quantity of an analyte present in a treatment area about the sensor, including but not limited to analytes such as of blood urea nitrogen, creatinine, fibrin, fibrinogen, immunoglobulins, deoxyribonucleic acids, ribonucleic acids, potassium, sodium, chloride, calcium, magnesium, lithium, hydronium, hydrogen phosphate, bicarbonate, and the like. However, many other analytes are also contemplated herein. Exemplary chemical/analyte sensors are disclosed in commonly owned U.S. Pat. No. 7,809,441 to Kane et al.

Although the physiological, respiration, or chemical sensors 516 are shown as part of a medical device in FIG. 5, it is realized that in some embodiments one or more of the physiological, respiration, or chemical sensors could be physically separate from the medical device. In various embodiments, one or more of the physiological, respiration, or chemical sensors can be within another implanted medical device communicatively coupled to a medical device via telemetry interface 518. In yet other embodiments, one or more of the physiological, respiration, or chemical sensors can be external to the body and coupled to a medical device via telemetry interface 518.

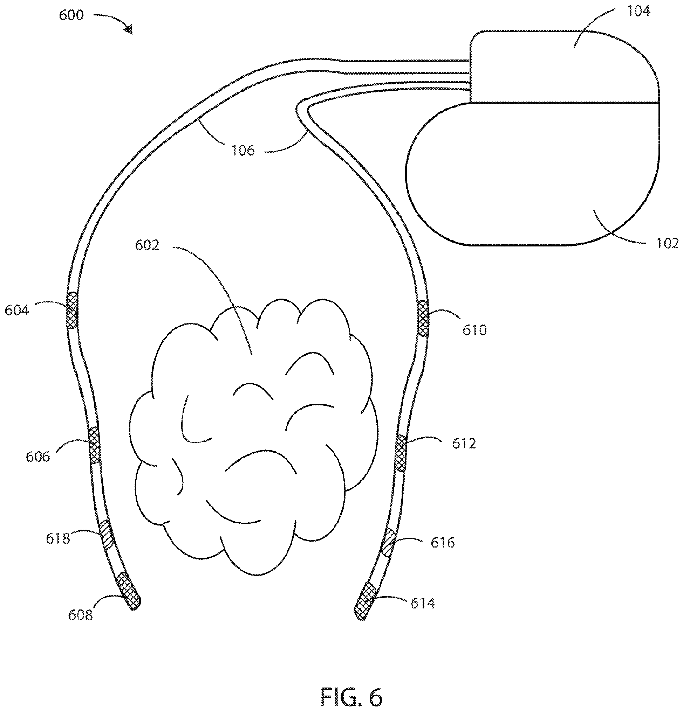

Referring now to FIG. 6, a schematic diagram of a medical device 600 is shown in accordance with the embodiments herein. Medical device 600 can include housing 102 and header 104, and one or more leads 106. Leads 106 can include one or more electrodes such as electrodes 604, 606, 608, 610, 612, or 614 disposed along the length of the leads 106. In some embodiments, electrodes 604, 606, 608, 610, 612, or 614 can include electric field generating electrodes and in other embodiments electrodes 604, 606, 608, 610, 612, or 614 can include electric field sensing electrodes. In some embodiments, leads 106 can include both electric field generating and electric field sensing electrodes.

The proximal ends of leads 106 are disposed within the header 104. The distal ends of electrical leads 106 can surround a cancerous tumor 602 such that the electrodes 604, 606, 608, 610, 612, or 614 are brought into proximity of the cancerous tumor 602. In some embodiments, the leads 106 can be positioned within the vasculature such that electrodes 604, 606, 608, 610, 612, or 614 are adjacent to or positioned within the cancerous tumor 602. However, it will be appreciated that leads 106 can be disposed in various places within or around the cancerous tumor 602. In some embodiments, the leads 106 can pass directly through the cancerous tumor 602.

In some embodiments, the leads 106 can include one or more tracking markers 616 or 618 along the length of the lead for use in determining the precise location of the electrodes relative to the tumor. In some embodiments, the one or more tracking markers can be disposed directly distal or directly proximal to the one or more electrodes disposed on the lead. In some embodiments, the tracking markers can be formed from a magnetic material. In some embodiments, the tracking markers can be formed from a radiographic material. In some embodiments, the tracking markers can be formed from a fluorographic material.

It will be appreciated that a plurality of electric field vectors can be generated between various combinations of electrodes 604, 606, 608, 610, 612, or 614 disposed along leads 106 to create an electric field. For example, one or more electric field vectors can be generated between electrodes 604 and 610. Similarly, one or more electric field vectors can be generated between electrodes 606 and 612. It will also be appreciated that one or more electric field vectors can be generated between any combination of electrodes 604, 606, 608, 610, 612, or 614. In some embodiments, one or more electric field vectors can be generated between any combination of electrodes 604, 606, 608, 610, 612, or 614 and the housing 102 of medical device 400. It will be appreciated that one or more unipolar or multipolar leads can be used in accordance with the embodiments herein. In some embodiments, a combination of unipolar and multipolar leads can be used. In other embodiments, a circular lead, clamp lead, cuff lead, paddle lead, or patch lead can be used.

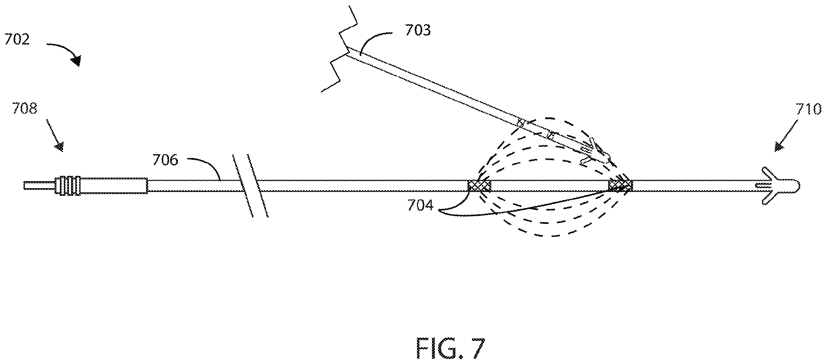

Referring now to FIG. 7, a lead 702 is shown in accordance with the embodiments herein. Lead 702 can include electrodes 704 disposed along the length of the lead 702. In some embodiments, electrodes 704 can be electric field generating electrodes. In some embodiments, electrodes 704 can be electric field sensing electrodes. In some embodiments, electrodes 704 can include a combination of electric field generating electrodes and electric field sensing electrodes. As discussed herein, electrodes can refer to either electric field generating electrodes or electric field sensing electrodes unless specifically described as one or the other. In some embodiments, lead 702 can include one or more dedicated impedance monitoring electrodes (not shown).

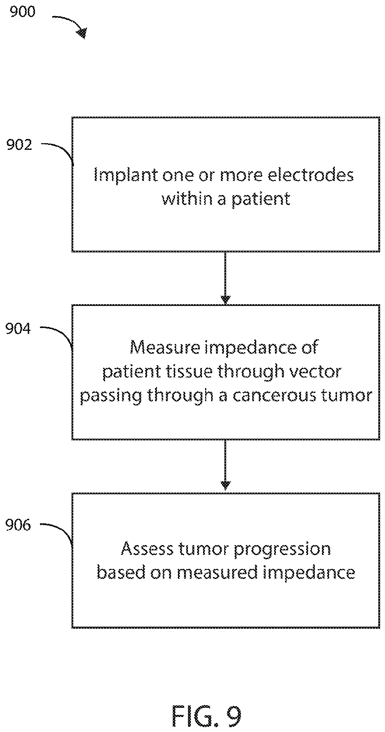

In some embodiments, impedance can be measured by taking the voltage dividing by the current. Within the body, impedance can be influenced by a number of factors, including but not limited to components in contact with an electric field such as cell type, including muscle, fat, connective tissue, and bone; cell density, cell size; electrolyte concentrations, etc. In some embodiments, electric field sensing or electric field generating electrodes can serve as impedance monitoring electrodes. It will be appreciated that different tissues will have different impedances at a given frequency. As such, in some embodiments, measuring impedance at one or more frequencies at any given location is contemplated and described in more detail below in reference to FIG. 9. In some embodiments, impedance can be measured at frequencies within the range of treatment frequencies. In some embodiments, impedance can be measured at frequencies outside of treatment frequencies. In some embodiments, impedance can be measured at both frequencies within the range of treatment frequencies and frequencies outside of treatment frequencies.

The lead 702 can include a lead body 706 having a proximal end 708 and a distal end 710. The lead body 706 can include one or more conductors (not shown) passing through the lead body 706 and providing electrical communication between the one or more electrodes 704 and the proximal end 708 of the lead body 706. One or more leads 702 can be implanted at the site of a cancerous tumor. In some embodiments, one or more leads 702 can be implanted within a cancerous tumor. Leads 702 can be disposed within the header of medical devices embodied herein and can be configured to be in electrical communication with control circuitry within the medical device.

In some embodiments, lead 702 can be configured by the electric field generating circuitry (not shown) to generate an electric field at the site of a cancerous tumor within the body of a patient. Lead 702 can be configured to generate an electric field from one or more electric field generating electrodes disposed thereon. In some embodiments, lead 702 can include both electric field generating electrodes and electric field sensing electrodes. In some embodiments, a separate lead 703 having electric field sensing electrodes disposed thereon can be implanted at or near the site of a cancerous tumor to serve as an electric field sensing lead. In some embodiments, a non-active electric field generating electrode on any of leads 702 or 703 and in proximity to a generated electric field can be configured to indirectly sense an electric field strength by measuring the voltage at that electric field generating electrode. It will be appreciated that a medical device as described here can be in electrical communication with the one or more electrodes 704 to serve itself as an electrode capable of generating an electric field.

Electric field sensing electrodes can be utilized to sense an electric field strength in or around a tumor during the course of any given therapy. Recorder circuitry within a medical device can be configured to record the data produced by the electric field sensing electrodes and the data can be used by a clinician to adjust the electric field strength to maintain a desired electric field strength at the site of the cancerous tumor. In some embodiments, the electric field generating circuit can be configured to adjust the electric field strength automatically in response to one or more data values sensed by electric field sensing electrodes to maintain a desired electric field strength at the site of the cancerous tumor. Suitable electric field strengths for use herein are described above in reference to FIG. 3 and the control circuitry 306 and the electric field generating circuit 320.

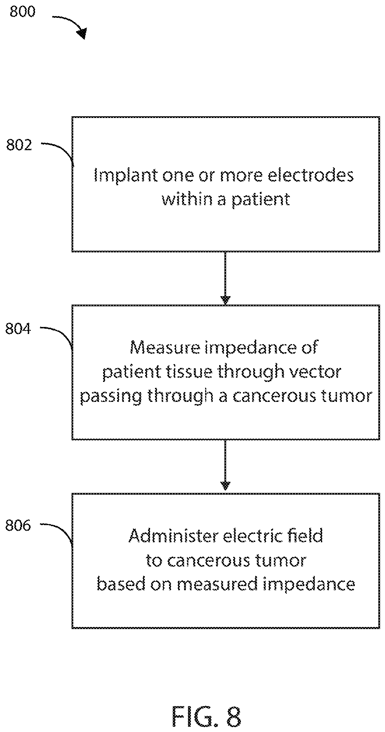

Referring now to FIG. 8, a method 800 for treating a cancerous tumor is shown. Method 800 can include implanting one or more electrodes within a patient at 802. Following implantation of one or more electrodes, the method 800 can include measuring the impedance of tissue within the patient along a vector passing through or near a cancerous tumor at 804. Method 800 can also include administering an electric field to the cancerous tumor of the patient based on the measured impedance at 806.

In some embodiments, method 800 can further include administering an electric field to a cancerous tumor of a patient based on changes in the measured impedance. Without being bound by any particular theory, it is believed that the impedance within a cancerous tumor is relatively low when compared to non-cancerous or necrotic tissue. This phenomenon allows impedance to be monitored as a function of therapy duration and to serve as a diagnostic tool in assessing whether or not a tumor is responding to an electric field therapy. If the impedance within a treatment area increases (across a fixed distance or area as a result of the low-impedance tumor tissue shrinking and non-cancerous tissue occupying the remaining space) then this can be taken as an indication that the electric field therapy is effectively decreasing the size of the cancerous tumor. However, if the impedance within a treatment area decreases or stays the same across a fixed distance or area then this can be taken as an indication that the electric field therapy is not decreasing the size of the cancerous tumor. As such, electric field therapies can be tailored to a particular cancerous tumor in order to effectively decrease the size of the cancerous tumor. By way of example, one or more of the amplitude, frequency, pulse width, waveform, directionality, and/or duty cycle of the electric field therapy can be modulated and/or changed.

The electrodes suitable for use in method 800 can include functionality allowing them to act as active electric field generating electrodes or passive electric field sensing electrodes. In some embodiments, the electric field generating electrodes can act as passive electric field sensing electrodes configured to measure voltage of an electric field at a given point in time. In some embodiments, passive electric field sensing electrodes can be implanted within the cancerous tumor. In some embodiments, passive electric field sensing electrodes can be implanted adjacent the cancerous tumor. In some embodiments, passive or active electrical field generating electrodes can implanted adjacent to the cancerous tumor. In various embodiments, a medical device including one or more components described with respect to FIGS. 3 and 5 can be configured to execute one or more operations described with respect to FIG. 8.

Referring now to FIG. 9, a method 900 for treating a cancerous tumor is shown. Method 900 can include implanting one or more electrodes within a patient at 902. Following implantation of one or more electrodes, the method 900 can include measuring the impedance of tissue within the patient along a vector passing through or near a cancerous tumor at 904. Method 900 can also include assessing tumor progression based on the measured impedance at 906. After assessing tumor progression, the method 900 can further include administering an electric field to a cancerous tumor of a patient based on the measured impedance. In some embodiments, an increased impedance across a fixed distance or area can be indicative of tumor regression. In other embodiments, a decrease in impedance can be indicative of tumor progression or resistance to electric field therapy.

Without being bound by any particular theory, it is believed that a cancerous tumor has a particular impedance associated therewith. In some embodiments, low-frequency impedance through a particular cancerous tumor can be used to measure conductivity through the tumor and can be used as an indicator of tissue progression or regression. In some embodiments, high-frequency impedance through a particular cancerous tumor can be used to measure permittivity and capacitive properties of the tumor and can also be used as an indicator of tissue progression or regression. In some embodiments, low-frequency impedance can be measured at frequencies of about 1 Hz to about 10 Hz. In some embodiments, high-frequency impedance can be measured at frequencies of about 10 Hz to about 1 Mz. In some embodiments, high-frequency impedance can be measured at frequencies of about 100 kHz to about 300 kHz. In various embodiments, a medical device including one or more components described with respect to FIGS. 3 and 5 can be configured to execute one or more operations described with respect to FIG. 9.

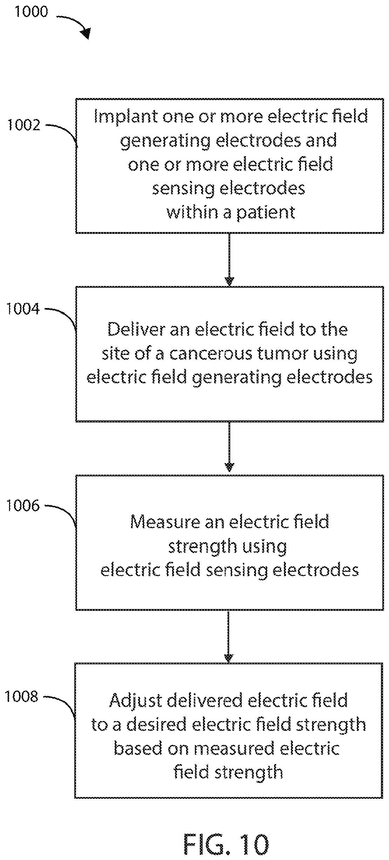

Referring now to FIG. 10, a method 1000 for treating a cancerous tumor is shown. Method 1000 can include implanting one or more electric field generating electrodes and one or more electric field sensing electrodes within a patient at 1002. Following implantation of the one or more electric field generating or electric field sensing electrodes, the method 1000 can include delivering an electric field from the electric field generating electrodes to a cancerous tumor at 1004. Method 1000 can include measuring an electric field strength with the electric field sensing electrodes in or around the cancerous tumor at 1006. Electric field strength can be measured in various ways, but in some embodiments, can include calculating electric field strength based on a measured voltage and known spatial separation between measuring electrodes. Method 1000 can further include adjusting the delivered electric field to a desired electric field strength based on the measured electrical field strength at 1008. In various embodiments, a medical device including one or more components described with respect to FIGS. 3 and 5 can be configured to execute one or more operations described with respect to FIG. 10.

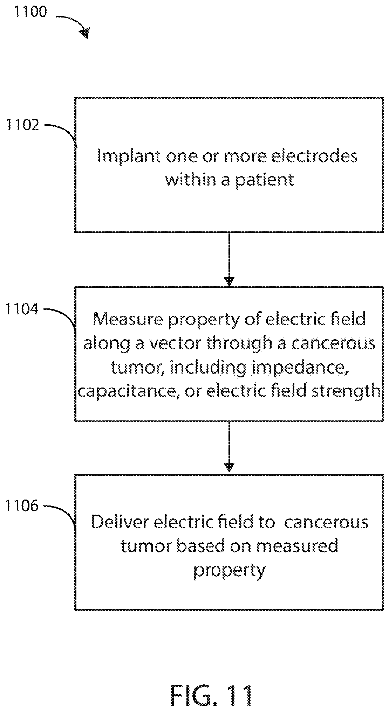

Referring now to FIG. 11, a method 1100 for treating a cancerous tumor is shown. Method 1100 can include implanting one or more electrodes within a patient at 1102. Method 1100 can further include measuring a property of an electric field delivered along a vector passing through or near a cancerous tumor, the property selected from the group consisting of impedance, capacitance, and electric field strength at 1104. Method 1100 can further include delivering an electric field to the cancerous tumor based on the measured property at 1106. In some embodiments, method 1100 can further adjusting the delivered electric field based on the measured property. Adjusting the delivered electric field can include one or more of changing the electric field strength, changing the waveform of the electric field, and changing the frequency of the electric field. In some embodiments, adjusting the delivered electric field can be performed automatically by the electric field generating circuit regardless of the local conditions, such as tumor progression or regression, local electrolyte concentrations, electrode state, electrode encapsulation, electrode impedance, tissue impedance, etc. In various embodiments, a medical device including one or more components described with respect to FIGS. 3 and 5 can be configured to execute one or more operations described with respect to FIG. 11.

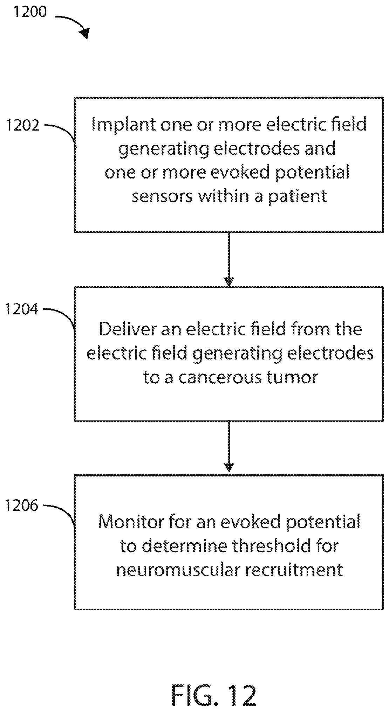

Referring now to FIG. 12, a method 1200 for treating a cancerous tumor is shown. Method 1200 can include implanting one or more electric field generating electrodes and one or more evoked potential sensors within a patient at 1202. In some embodiments, an evoked potential sensor can be a sensor configured to detect one or more electrical potentials emitted by the nervous system, such as from the cerebral cortex, the brain stem, the spinal cord, and the peripheral nerves. In some embodiments, an evoked potential sensor can be a sensor configured to detect one or more electrical potentials emitted other electrical sources in the body such as from cardiac or skeletal muscle. In some embodiments, the electric field sensing and electric field generating electrodes can be configured to act as an evoked potential sensor. The evoked potential sensor can include electrodes (such as electrode on leads described herein) in order to measure electrical properties such as electric potential, current, and/or impedance.

Method 1200 can further include delivering an electric field from the electric field generating electrodes to a cancerous tumor at 1204. The evoked potential sensor can be used for monitoring for an evoked potential response. In some embodiments, the evoked potential sensor can be used to determine whether or not the delivered electric field strength is above a predetermined threshold for neural or muscular recruitment at 1206. In some embodiments, a predetermined threshold can be determined prior to treatment. In some embodiments, the delivered electric field strength can be titrated until an evoked potential response is observed by the evoked potential sensor, and the electric field strength can be turned down so as not to generate and evoked potential response by neural or muscular tissues. Titrating the electric field strength can be performed manually by a clinician or it can be performed automatically such as the titration process being programmed into the memory of and controlled by the medical device. The method 1200 can further include reducing the strength of the electric field if the electric field strength is above a threshold for neural or muscular recruitment. In some embodiments, the electric field strength can be empirically determined so as to reach an electric field strength that is as high as possible before leading to neural or muscular recruitment. In various embodiments, a medical device including one or more components described with respect to FIGS. 3 and 5 can be configured to execute one or more operations described with respect to FIG. 12.

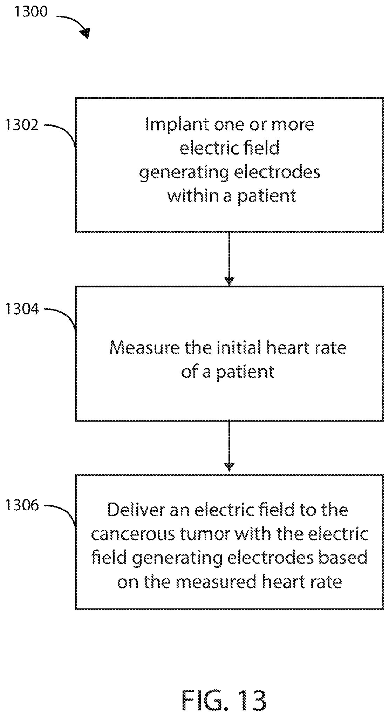

Referring now to FIG. 13, a method 1300 for treating a cancerous tumor is shown. Method 1300 can include implanting one or more electric field generating electrodes within a patient at 1302. Method 1300 can also include measuring an initial heart rate of a patient at 1304. An electric field can be delivered from the electric field generating electrodes to a cancerous tumor at 1306. The method 1300 can include monitoring for changes in the heart rate of the patient in response to the delivered electric field at 1306. The method 1300 can also include adjusting the electric field if changes in the heart rate are detected 1308. In some embodiments, adjusting the electric field can include reducing the strength of the delivered electric field to a predetermined threshold that does not induce a change in a patient's heart rate. In some embodiments, adjusting the electric field can include increasing the strength of the delivered electric field to a predetermined threshold just prior to detecting a change in a patient's heart rate. In various embodiments, a medical device including one or more components described with respect to FIGS. 3 and 5 can be configured to execute one or more operations described with respect to FIG. 13.

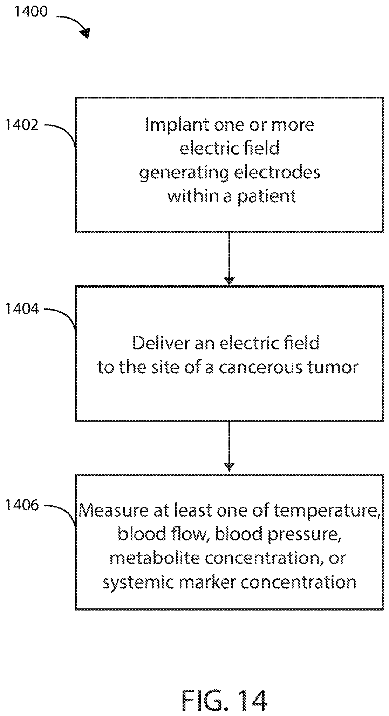

Referring now to FIG. 14, a method 1400 for treating a cancerous tumor is shown. Method 1400 can include implanting one or more electric field generating electrodes within a patient at 1402. Method 1400 can include delivering an electric field from the electric field generating electrodes to a cancerous tumor at 1404. The method 1400 can include measuring at least one property selected from the group consisting of temperature, blood flow, blood pressure, metabolite concentrations, and systemic cancer markers at 1406.

Temperature, blood flow, and blood pressure can be monitored by temperature and pressure sensors mounted within or near a lead positioned at or within a cancerous tumor. Temperature and pressure sensors can be configured to monitor temperature, blood flow, and blood pressure at or near the site of the cancerous tumor. Exemplary transthoracic impedance sensors capable of monitoring blood flow, heart rate, blood pressure and the like are described in commonly owned U.S. Pat. No. 8,795,189. Exemplary chemical metabolites, or analytes, and methods of sensing the same are disclosed in commonly owned U.S. Pat. No. 7,809,441 to Kane et al.

Exemplary systemic cancer markers can include, but are not limited to, ALK gene overexpression, alpha-fetoprotein (AFP), beta-2-microglobulin (B2M), beta-human chorionic gonadaotropin (beta-hCG), BRCA1 and BRCA2 gene mutations, BCR-ABL gen fusion, BRAF V600 mutations, BUN/creatinine, CD117/C-kit, CA15-3/CA27.29, CA19-9, CA-125, calcitonin, carcinoembryonic antigen (CEA), CD20, chromogranin A (CgA), cytokeratin fragment 21-1, epidural growth factor receptor (eGFR) gene mutations, estrogen receptor (ER) and progesterone receptor (PR) gene mutations, fibrin and fibrinogen, HE4, HER2/neu gene and protein upregulation, immunoglobulins, KRAS gene mutations, lactate dehydrogenase, non-specific enolase (NSE), nuclear matrix protein 22, programmed cell death ligand 1 (PD-L1), prostate-specific antigen (PSA), thyroglobulin, urokinase plasminogen activator (uPA) and plasminogen activator inhibitor (PAI-1), and the like.

Systemic cancer markers can be monitored by removal of a sample of tissue or bodily fluid from within or near the site of a cancerous tumor. In some embodiments, the leads described herein can include an open lumen that runs the entire longitudinal length of, or a select portion of the longitudinal length of the lead. The open lumen can include an integrated biopsy apparatus suitable for obtaining biopsy samples from a cancerous tumor site on a periodic basis to monitor disease progression and/or regression by analyzing the tissue or fluid for one or more systemic cancer markers.

In some embodiments, method 1400 can include monitoring for changes in the at least one property. If changes in the at least one property are detected, the method 1400 can also include altering the delivered electric field based on the measured values of the at least one property. In various embodiments, a medical device including one or more components described with respect to FIGS. 3 and 5 can be configured to execute one or more operations described with respect to FIG. 14.

### Leads and Electrodes

The leads described herein can be placed into the body near the site of a cancerous tumor using a number of techniques. Placement of one or more leads can include using techniques such as transvascular placement, tunneling into the subcutaneous space, and/or surgical placement. In some embodiments, the placement of one or more leads can include placement via one or more natural body orifices. The leads can be placed adjacent to or within a cancerous tumor. In some embodiments, multiple leads can be used near to or far from the cancerous tumor.

In some embodiments one or more leads described herein can be placed in the subcutaneous space. Electrodes on leads placed in the subcutaneous space can be used as the primary near-field generating electrode or as a far-field field generating electrode. In some embodiments, electrodes on leads placed in the subcutaneous space can be used as the primary near-field generating electrode or as a far-field field generating electrode in conjunction with the housing of a medical device. Likewise, one or more leads can be placed transvascularly to act as far-field field generating electrodes in conjunction with an electrode at or near the site of the cancerous tumor or in conjunction with the housing of a medical device.

The leads and electrodes described herein can include additional functional and structural features. In some embodiments, the leads can include those that are compatible with imaging and treatment techniques, including but not limited to MRI (magnetic resonance imaging), X-ray imaging, deep brain stimulation techniques, and/or radiation therapy. In some embodiments, the leads can include one or more conductor cores made from conducting materials. The conductor cores can be formed from conducting materials including metals and/or other conducting materials. Metals can include, but are not limited to, palladium, platinum, silver, gold, copper, aluminum, various alloys including stainless steel, nickel-cobalt alloys such as MP35N^{®} and the like. In some embodiments, the conductor core can be a multifilar coil, including but not limited to a bifilar coil, a trifilar coil, and a quadfilar coil.

In some embodiments, electrodes can be disposed along the length of one or more leads as described herein. Suitable materials for use in the electrodes described herein can include metals such as palladium, to minimize coupling and artifact generation in magnetic fields. In some embodiments, electrodes can be made from other metals and/or other conducting materials. Metals can include, but are not limited to, palladium, platinum, platinum alloys such as platinum-iridium alloy, gold, copper, tantalum, titanium, various alloys including stainless steel, and the like. In some embodiments, electrodes can be in the form of wound coils that can provide an added benefit of increased surface area without compromising flexibility of the electrodes. In some embodiments, the implantable device housing can serve as an electrode.

The leads described herein can also include one or more electrodes disposed along the length of the lead. The leads can include two or more electrodes disposed along the length of the lead. In some embodiments, the electrodes can be tip electrodes found at the distal end of the lead. In other embodiments, the electrodes can be ring electrodes found along the lead but not at the tip of the lead. In some embodiments, the electrodes can be coil electrodes. In some embodiments, a ring or tip electrode can be positioned in or adjacent to a tumor or cancerous tissue and a coil electrode can be positioned farther from the tumor or cancerous tissue in order to help provide spatial diversity to the generated electric fields. In some embodiments, one or more electrodes can have a length along the lengthwise axis (e.g., proximal to distal axis) of about 0.5, 1, 1.5, 2, 3, 4, 5, 7.5, 10, 15, 20, 30, 40, 50, 75, 100 mm or more. In some embodiments, one or more of the electrodes can have a length falling within a range wherein any of the foregoing distances can serve as the upper or lower bound of the range, provided that the upper bound is greater than the lower bound.

The leads can be unipolar, bipolar, or multipolar. In some embodiments, a unipolar lead can include a lead that generates an electric field between one electrode and the housing of the medical device. In some embodiments, a bipolar lead can include a lead that can generate and electric field between two electrodes disposed along the lead, or between both electrodes and the housing of the medical device. In some embodiments, a multipolar lead can include a lead that can generate an electric field between the more than two electrodes disposed along the lead, between more than two electrodes and the housing of the medical device, or any number of combinations of configurations of electrodes and the housing of the medical device.

The electrodes suitable for use here can be made of conductive polymers such as carbon filled silicone, polyacetylene, polypyrrole, polyaniline, polytiophene, polyfuran, polyisoprene, polybutadiene, polyparaphenylene, and the like. In other embodiments, the electrodes can be insulated. In some embodiments, the insulation surrounding and electrode can include microporous insulators to prevent cellular apposition, yet still allow for current flow. Microporous insulators can be made from a number of the insulating materials described herein, including but not limited to polytetrafluoroethylene (ePTFE), polyethylene-co-tetrafluoroethene (ETFE), polyurethanes, silicones, poly(p-xylylene) polymers such as Parylene polymers, polyether block amides such as PEBAX^{®}, nylons, or derivatives thereof. In some embodiments, the electrodes can be coated with various materials, including but not limited to hydrogels or fractal coatings such as iridium oxide, titanium oxide, tantalum pentoxide, other metal oxides, poly(p-xylylene) polymers such as Parylene, and the like.

A number of lead fixation techniques and configurations can be used in accordance with the embodiments herein. Some non-limiting examples of lead fixation techniques can include biocompatible glue fixation, talon fixation, helix coil fixation, passive centering of the lead in the vascular system, tine fixation within the localized vascular system, spiral bias fixation within the localized vascular system, compression fixation, suture sleeve fixation, and the like. In some examples, the leads embodied herein can be placed within the vascular system surrounding or adjacent to the site of the cancerous tumor. In other embodiments, the leads embodied herein can be place surgically at or within or surrounding the site of the cancerous tumor.

The leads suitable for use herein can also include one or more open lumens that run the entire longitudinal length of, or a select portion of the longitudinal length of the lead. In some embodiments, the open lumen can include an integrated biopsy apparatus suitable for obtaining biopsy samples from a cancerous tumor site on a periodic basis to monitor disease progression and/or regression. Leads having an open lumen can also be configured to include an integrated drug delivery lumen that can deliver one or more drugs, such as steroids or chemotherapy agents, to the site of the tumor in a single bolus or periodically via a metered pump. The leads can include one or more portals disposed along the length of the lead to provide an outlet for drug delivery at or near the site of a cancerous tumor.

In some embodiments a portion of the lead or the entire lead can include a drug eluting coating. In some embodiments, the drug eluting coating can include an anti-inflammatory agent, such as a steroid. In some embodiments, the steroid can be dexamethasone. In other embodiments, the drug eluting coating can include a chemotherapy agent. In some embodiments, the chemotherapy agent can include a taxane or derivatives thereof, including but not limited to paclitaxel, docetaxel, and the like. In other embodiments, the drug eluting coating can be configured to release additional classes of chemotherapy agents, including, but not limited to alkylating agents, plant alkaloids such as vinca alkaloids, cytotoxic antibiotics, topoisomerase inhibitors, and the like. In some embodiments, the drug eluting coating can be configured to release the drug from the coating in a time-release fashion.

The leads herein can adopt a number of shapes or configurations. In some embodiments, the leads can be linear and in other embodiments the leads can be circular. A circular lead may be a completely closed loop or it may be a semi-closed loop. In some embodiments, the lead can include a bendable core that can allow the lead to be shaped into many configurations, including but not limited to a U shape, an S shape, a spiral shape, a half circle, an oval, and the like.

In yet other examples, the leads suitable for use herein can include fluorimetric or magnetic markers that can assist the clinician in precise placement at or near the site of a cancerous tumor. The leads can also include integrated pH sensors for detecting the change in the pH at or near the cancerous tumor or other chemical sensors suitable for analyzing the concentration of a chemical analyte of interest.

### Therapy Parameters

Successful treatment of cancerous tumors can depend on a number of variables, including electric field strength, frequency, cell heterogeneity, cell size, cancer cell type, tumor size, and location within the body. A variety of therapy parameters can be implemented using the medical devices described herein. One or more therapeutic parameter sets can be programmed into the memory of the medical devices and implemented by the control circuitry 306, shown in FIG. 3. Exemplary therapeutic parameter sets can include those that implement the following concepts: sweeping through a range of frequencies; stacking of one or more frequencies simultaneously; stepping through one or more frequencies sequentially; the spatial or temporal delivery of one or more electric fields; sweeping through a range of electric field strengths; applying an effective spinning electric field; modulating a voltage control mode or a current control mode; implementing one or more duty cycles; pulse width modulation; manipulation of the waveform shape and/or pulse sequence; and the occasional use of high frequency or high electric fields strength pulses.

The therapeutic parameter sets can be programmed into a medical device to operate autonomously, or they can be queried and manipulated by the patient or a clinician using an external computation device such as a programmer, a home-based unit, and/or a mobile unit (e.g. a cellular phone, personal computer, smart phone, tablet computer, and the like). In other embodiments, the therapeutic parameter sets can be wirelessly communicated to the medical device from an external computation device. Frequencies and/or electric field strengths suitable for use in any of the therapeutic parameter sets herein are discussed above with respect to electric field generating circuit 320. In some embodiments, one or more therapeutic parameter sets can be implemented simultaneously. In other embodiments, one or more therapeutic parameter sets can be implemented in an alternating fashion.

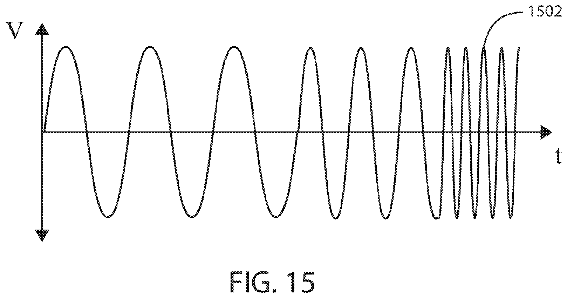

Referring now to FIG. 15, exemplary plot 1502 shows an example of sweeping through a range of frequencies at the site of a cancerous tumor. Plot 1502 shows an alternating electric field, where the frequency is increased over time as the therapy is applied to the cancerous tumor. In some embodiments, a frequency sweep can include alternating between a first frequency sweep covering a range of about 100 kHz to 300 kHz and a second frequency sweep covering a range about 200 kHz to 500 kHz. It will be appreciated that sweeping through a first and second frequency range as described can be performed indefinitely throughout the course of the therapy.

### Electric Field Generators

The medical devices embodied herein can include electric field generators particularly suited for therapeutic and diagnostic techniques used during the course of treatment for a cancerous tumor. In some embodiments, the electric field generators suitable for use herein can include those that have been treated by radiation hardening to make the components resistant to the damaging effects of radiation therapy treatments often prescribed as a main line treatment for cancerous tumors. Electric field generators can include components such as those described in reference to FIGS. 3 and 5 above.

Electric field generators embodied herein can be programmed with any number of therapeutic parameter sets as described. The electric field generators can be programmed prior to implant, or they can be programmed by a clinician using an external computation device such as a programmer, a home-based unit, and/or a mobile unit (e.g. a cellular phone, personal computer, smart phone, tablet computer, and the like). In some embodiments, therapy parameters can be delivered to the electric field generator via a telemetry circuit. In some embodiments, the electric field generator can include a recharge circuit communicatively coupled to a receiver coil to facilitate transcutaneous recharging of the medical device. In some embodiments, the electric field generator can communicate wirelessly between the receiver coil and an external charging device.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration to. The phrase "configured" can be used interchangeably with other similar phrases such as arranged and configured, constructed and arranged, constructed, manufactured and arranged, and the like.

All publications and patent applications in this specification are indicative of the level of ordinary skill in the art to which this invention pertains.

Aspects have been described with reference to various specific and preferred embodiments and techniques. However, it should be understood that many variations and modifications may be made while remaining within the scope herein. The scope of the claimed invention is defined by the appended set of claims.

## Claims

1. An implantable medical device (100; 200; 600) for treating a cancerous tumor of a patient, comprising:
a first and a second implantable lead (106, 702), both leads including one or more electric field generating electrodes (512; 604, 606, 608; 610, 612, 614; 704) and one or more impedance monitoring electrodes (508; 604, 606, 608; 610, 612, 614; 704) configured to measure the impedance of tissue within the patient along a vector passing through the cancerous tumor;
wherein the medical device is configured to generate an electric field at frequencies selected from a range of between 10 kHz to 1 MHz, wherein the electric field is effective to disrupt cellular mitosis in cancerous cells,
wherein the electric field generating electrodes are configured to administer the electric field to the cancerous tumor of the patient based on the measured impedance; and
wherein the medical device is configured to administer the electric field to the cancerous tumor of the patient based on changes in the measured impedance such that the medical device is configured to adjust the electric field, including changing one or more of the amplitude, frequency, pulse width, wave form, directionality, and/or duty cycle, in response to a measured decrease in impedance reflecting a lack of a decrease in the size of the tumor.

2. The medical device of claim 1, the impedance monitoring electrodes comprising passive electric field sensing electrodes (508; 604, 606, 608; 610, 612, 614; 704).

3. The medical device of any of claims 1-2, wherein the passive electric field sensing electrodes are configured to be implanted within the cancerous tumor.

4. The medical device of any of claims 1-3, further comprising one or more transthoracic impedance sensors configured to monitor for changes in the heart rate of the patient in response to the delivered electric field,
wherein the medical device is configured to adjust the electric field if changes in the heart rate are detected.

5. The medical device of claim 4, wherein the medical device is configured such that adjusting the electric field comprises reducing the strength of the delivered electric field to a predetermined threshold.

6. The medical device of any of claims 1-5, further comprising
one or more temperature or pressure sensors (516) configured to measure at least one property selected from the group consisting of temperature, blood flow, blood pressure; and
wherein the medical device is configured to monitor for changes in the at least one property.

7. The medical device of any of claims 1-6,
further configured to assess tumor progression based on the measured impedance.

8. The medical device of claim 7, the measured impedance comprising a measured low frequency impedance.

9. The medical device of claim 8, the low frequency comprising a frequency of about 1 Hz to about 10 Hz.

10. The medical device of any of claims 7-9, the measured impedance comprising a measured high frequency impedance.

11. The medical device of claim 10, the high frequency comprising a frequency of about 100 kHz to about 300 kHz.

12. The medical device of any one of the preceding claims,
the medical device being configured to:
measure an electric field strength with electric field sensing electrodes in or around the cancerous tumor; and
adjust the delivered electric field to a desired electric field strength based on the measured electrical field strength.

13. The medical device of any one of the preceding claims, wherein the electric field is administered along at least two vectors spatially separated by at least 10 degrees.

## Patentansprüche

1. Implantierbares medizinisches Gerät (100; 200; 600) zur Behandlung eines Krebstumors eines Patienten, aufweisend:
eine erste und eine zweite implantierbare Leitung (106, 702), wobei beide Leitungen eine oder mehrere ein elektrisches Feld erzeugende Elektroden (512; 604, 606, 608; 610, 612, 614; 704) beinhalten, und eine oder mehrere Impedanz überwachende Elektroden (508; 604, 606, 608; 610, 612, 614; 704), die konfiguriert sind, die Impedanz von Gewebe innerhalb des Patienten entlang eines Vektors zu messen, der durch den Krebstumor hindurchläuft;
wobei das medizinische Gerät konfiguriert ist, ein elektrisches Feld bei Frequenzen zu erzeugen, die aus einem Bereich zwischen 10 kHz bis 1 MHz ausgewählt sind, wobei das elektrische Feld wirksam ist, Zellmitose in Krebszellen zu unterbrechen,
wobei die ein elektrisches Feld erzeugenden Elektroden konfiguriert sind, das elektrische Feld basierend auf der gemessenen Impedanz auf den Krebstumor des Patienten anzuwenden; und
wobei das medizinische Gerät konfiguriert ist, das elektrische Feld basierend auf Änderungen der gemessenen Impedanz auf den Krebstumor des Patienten so anzuwenden, dass das medizinische Gerät konfiguriert ist, das elektrische Feld als Reaktion auf eine gemessene Impedanzabnahme, welche eine fehlende Abnahme der Größe des Tumors widerspiegelt, anzupassen, beinhaltend ein Ändern von einem oder mehreren aus der Amplitude, Frequenz, Pulsbreite, Wellenform, Direktionalität und/oder Tastverhältnis.

2. Medizinisches Gerät nach Anspruch 1, wobei die Impedanz überwachenden Elektroden passive, ein elektrisches Feld erfassende Elektroden (508; 604, 606, 608; 610, 612, 614; 704) aufweisen.

3. Medizinisches Gerät nach irgendeinem der Ansprüche 1-2, wobei die passiven, ein elektrisches Feld erfassenden Elektroden konfiguriert sind, innerhalb des Krebstumors implantiert zu werden.

4. Medizinisches Gerät nach irgendeinem der Ansprüche 1-3, ferner aufweisend einen oder mehrere transthorakale Impedanzsensoren, die konfiguriert sind, Änderungen der Herzfrequenz des Patienten als Reaktion auf das abgegebene elektrische Feld zu überwachen,
wobei das medizinische Gerät konfiguriert ist, das elektrische Feld anzupassen, wenn Änderungen der Herzfrequenz detektiert werden.

5. Medizinisches Gerät nach Anspruch 4, wobei das medizinische Gerät so konfiguriert ist, dass das Anpassen des elektrischen Felds ein Verringern der Stärke des abgegebenen elektrischen Felds auf einen vorbestimmten Schwellwert umfasst.

6. Medizinisches Gerät nach irgendeinem der Ansprüche 1-5, ferner aufweisend:
einen oder mehrere Temperatur- oder Drucksensoren (516), die konfiguriert sind, zumindest eine Eigenschaft zu messen, die aus der Gruppe ausgewählt ist, die aus Temperatur, Blutfluss und Blutdruck besteht; und
wobei das medizinische Gerät konfiguriert ist, Änderungen der zumindest einen Eigenschaft zu überwachen.

7. Medizinisches Gerät nach irgendeinem der Ansprüche 1-6, ferner dafür konfiguriert, eine Tumorprogression basierend auf der gemessenen Impedanz zu bewerten.

8. Medizinisches Gerät nach Anspruch 7, wobei die gemessene Impedanz eine gemessene Niederfrequenzimpedanz umfasst.

9. Medizinisches Gerät nach Anspruch 8, wobei die Niederfrequenz eine Frequenz von etwa 1 Hz bis etwa 10 Hz umfasst.

10. Medizinisches Gerät nach irgendeinem der Ansprüche 7-9, wobei die gemessene Impedanz eine gemessene Hochfrequenzimpedanz umfasst.

11. Medizinisches Gerät nach Anspruch 10, wobei die Hochfrequenz eine Frequenz von etwa 100 kHz bis etwa 300 kHz umfasst.

12. Medizinisches Gerät nach irgendeinem der vorhergehenden Ansprüche, wobei das medizinische Gerät konfiguriert ist, um:
eine elektrische Feldstärke mit ein elektrisches Feld erfassenden Elektroden im Krebstumor oder um ihn herum zu messen; und
das abgegebene elektrische Feld basierend auf der gemessenen elektrischen Feldstärke auf eine gewünschte elektrische Feldstärke anzupassen.

13. Medizinisches Gerät nach irgendeinem der vorhergehenden Ansprüche, wobei das elektrische Feld entlang zumindest zweier Vektoren angewendet wird, die um mindestens 10 Grad räumlich voneinander getrennt sind.

## Revendications

1. Dispositif médical implantable (100 ; 200 ; 600) servant à traiter une tumeur cancéreuse d'un patient, comprenant :
un premier et un deuxième fils implantables (106, 702), les deux fils incluant une ou plusieurs électrodes générant un champ électrique (512 ; 604, 606, 608 ; 610, 612, 614 ; 704) et une ou plusieurs électrodes de surveillance d'impédance (508 ; 604, 606, 608 ; 610, 612, 614 ; 704) configurées pour mesurer l'impédance d'un tissu à l'intérieur du patient le long d'un vecteur passant à travers la tumeur cancéreuse ;
dans lequel le dispositif médical est configuré pour générer un champ électrique à des fréquences sélectionnées dans une plage comprise entre 10 kHz et 1 MHz, dans lequel le champ électrique est efficace pour interrompre la mitose cellulaire dans les cellules cancéreuses,
dans lequel les électrodes générant le champ électrique sont configurées pour administrer le champ électrique à la tumeur cancéreuse du patient sur la base de l'impédance mesurée ; et
dans lequel le dispositif médical est configuré pour administrer le champ électrique à la tumeur cancéreuse du patient sur la base de changements de l'impédance mesurée de sorte que le dispositif médical est configuré pour ajuster le champ électrique, incluant le changement d'un ou plusieurs parmi amplitude, fréquence, largeur d'impulsion, forme d'onde, orientation et/ou cycle de service, en réponse à une diminution mesurée de l'impédance reflétant une absence de diminution de la taille de la tumeur.

2. Dispositif médical selon la revendication 1, les électrodes de surveillance d'impédance comprenant des électrodes passives de détection de champ électrique (508 ; 604, 606, 608 ; 610, 612, 614 ; 704).

3. Dispositif médical selon l'une quelconque des revendications 1 et 2, dans lequel les électrodes passives de détection de champ électrique sont configurées pour être implantées à l'intérieur de la tumeur cancéreuse.

4. Dispositif médical selon l'une quelconque des revendications 1 à 3, comprenant en outre un ou plusieurs capteurs d'impédance transthoraciques configurés pour surveiller des changements de la fréquence cardiaque du patient en réponse au champ électrique délivré,
dans lequel le dispositif médical est configuré pour ajuster le champ électrique si des changements de la fréquence cardiaque sont détectés.

5. Dispositif médical selon la revendication 4, dans lequel le dispositif médical est configuré de sorte que l'ajustement du champ électrique comprend la réduction de l'intensité du champ électrique délivré à un seuil prédéterminé.

6. Dispositif médical selon l'une quelconque des revendications 1 à 5, comprenant en outre
un ou plusieurs capteurs de température ou de pression (516) configurés pour mesurer au moins une propriété sélectionnée dans le groupe consistant en température, débit sanguin, pression artérielle ; et
dans lequel le dispositif médical est configuré pour surveiller des changements de l'au moins une propriété.

7. Dispositif médical selon l'une quelconque des revendications 1 à 6,
configuré en outre pour évaluer la progression de la tumeur sur la base de l'impédance mesurée.

8. Dispositif médical selon la revendication 7, l'impédance mesurée comprenant une impédance basse fréquence mesurée.

9. Dispositif médical selon la revendication 8, la basse fréquence comprenant une fréquence d'environ 1 Hz à environ 10 Hz.

10. Dispositif médical selon l'une quelconque des revendications 7 à 9, l'impédance mesurée comprenant une impédance haute fréquence mesurée.

11. Dispositif médical selon la revendication 10, la haute fréquence comprenant une fréquence d'environ 100 kHz à environ 300 kHz.

12. Dispositif médical selon l'une quelconque des revendications précédentes, le dispositif médical étant configuré pour
mesurer une intensité de champ électrique avec des électrodes de détection de champ électrique dans ou autour de la tumeur cancéreuse ; et
ajuster le champ électrique délivré à une intensité de champ électrique souhaitée sur la base de l'intensité de champ électrique mesurée.

13. Dispositif médical selon l'une quelconque des revendications précédentes, dans lequel le champ électrique est administré le long d'au moins deux vecteurs séparés dans l'espace d'au moins 10 degrés.
